(19) Europäisches Patentamt
European Patent Office
Office européen des brevets

(11) Veröffentlichungsnummer: **0 003 988**
**B1**

# (12) EUROPÄISCHE PATENTSCHRIFT

(21) Anmeldenummer: **79100489.8**

(22) Anmeldetag: **20.02.79**

(51) Int. Cl.$^{3}$: **C 07 C 125/06**

## (54) Verfahren zur Herstellung von Urethanen

(30) Priorität: **02.03.78 DE 2808990**

(43) Veröffentlichungstag der Anmeldung:
**19.09.79 Patentblatt 79/19**

(45) Bekanntmachung des Hinweises auf die Patenterteilung:
**01.10.80 Patentblatt 80/20**

(84) Benannte Vertragsstaaten:
**BE DE FR GB IT NL**

(56) Entgegenhaltungen:
**DE - A - 2 343 826**
**DE - A - 2 614 101**
**DE - A - 2 623 694**

(73) Patentinhaber: **BAYER AG Zentralbereich Patente Marken und Lizenzen**
**D - 5090 Leverkusen 1,**
**Bayerwerk (DE)**

(72) Erfinder: **Scholl, Hans-Joachim, Dr.**
**Rudolf-Sohm-Strasse 28**
**D - 5000 Köln 60 (DE)**
**Zenner, Armin, Dr. Goethestrasse 65**
**D - 4047 Dormagen 1 (DE)**

**EP 0 003 988 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Urethanen

Die vorliegende Erfindung betrifft ein verbessertes Verfahren zur Herstellung von Urethanen durch Umsetzung von aromatischen Nitroverbindungen mit Alkoholen und Kohlenmonoxid in Gegenwart von Selen enthaltenden Katalysatorsystemen. Aus den genannten Ausgangsmaterialien zugängliche Urethane wurden bisher im allgemeinen durch Umsetzung eines aromatischen Isocyanats mit einem Alkohol gebildet, wobei das Isocyanat seinerseits durch Umsetzung von Phosgen mit dem entsprechenden primären Amin gewonnen wird, welches im allgemeinen wiederum durch Reduktion der entsprechenden Nitroverbindung erhalten worden war. Dieses herkömmliche Verfahren weist jedoch verschiedene Nachteile auf, nicht zuletzt aufgrund der Toxizität und der korrosiven Natur von Phosgen und der Bildung von Chlorwasserstoff als Nebenprodukt. Außerdem ist es bekannt, daß bestimmte aromatische Amine schädliche biologische Eigenschaften aufweisen und einige auch dazu neigen, bei der Lagerung durch Luft oxydiert zu werden.

Es mangelte daher auch nicht an Versuchen, den Umweg über das hochtoxische Phosgen zu vermeiden und die Urethane direkt aus den entsprechenden Nitroverbindungen und den entsprechenden Alkoholen sowie Kohlenmonoxid herzustellen. Die Verfahren der US-PS 3 993 685 bzw. der DE-OS 2 603 574 bedienten sich hierbei Katalysatorsysteme auf Basis von Metallen der Platin-Gruppe. Da bei diesen Verfahren merkliche Verluste der sehr kostspieligen Katalysatoren nicht vermieden werden können, fanden diese Verfahren bislang keinen Eingang in die großtechnische Praxis.

Beim Verfahren der DE-OS 2 343 826 wird als katalytisch wirkendes System eine Kombination aus Selen oder Schwefel bzw. Verbindungen dieser Elemente mit sehr großen Mengen einer Base vorgeschlagen. Als Basen kommen z.B. Triäthylamin und Pyridin in Frage. Um aber die Reaktion in Anwesenheit dieser Amine zufriedenstellend einzuleiten, erscheint es erforderlich, die Amine in einer ziemlich großen Menge im Hinblick auf die Ausgangnitroverbindung zu verwenden. In der Tat wird, wenn Dinitrotoluol als Nitroverbindung verwendet wird, das Amin in einer Menge verwendet, die gleich oder größer ist als diejenige des Dinitrotoluols. Die Verwendung derart großer Mengen an Amin bringt zahlreiche Probleme wirtschaftlicher Art und auch hinsichtlich der Rückgewinnungsverfahren mit sich. Weiterhin führt dieses Verfahren zur Bildung von Nebenprodukten, z.B. Aminoverbindungen und Harnstoffen, wenn meßbare Anteile an Wasser, z.B. als Hydrate, aber auch in freier Form vorliegen. Auch das Verfahren der DE-OS 2 343 826 ist daher für eine großtechnische Anwendung weitgehend ungeeignet.

Die wegen der genannten Bildung von Nebenprodukten herabgesetzte Ausbeute an den angestrebten Urethanen läßt sich beim Verfahren der DE-OS 2 614 101 durch ein Katalysator-System vermeiden, welches aus elementarem Selen bzw. einer Selenverbindung und einem Promotor, bestehend z.B. aus einem bicyclischen Amidin und einer Carbonsäure zusammengesetzt ist. Zwar sind beim Verfahren der DE-OS 2 614 101 die Ausbeuten an Urethanen im Vergleich zum Verfahren der DE-OS 2 343 826 erhöht, jedoch entstehen auch hierbei störende Mengen an Nebenprodukten, welche insbesondere Hydrolyse- und Folgeprodukte des gebildeten Urethans darstellen.

Das Verfahren der DE-OS 2 623 694 muß insofern als eine Weiterenwicklung des Verfahrens der DE-OS 2 614 101 betrachtet werden, als die Bildung der Nebenprodukte aus den Urethanen durch Mitverwendung von diesen Nebenprodukten entsprechenden aromatischen Aminoverbindungen bzw. aromatischen Harnstoffverbindungen zurückgedrängt wird. Obwohl mit dieser Maßnahme eine Verbesserung des Verfahrens gemäß DE-OS 2 614 101 möglich ist, ist auch das Verfahren der DE-OS 2 623 694 noch mit schwerwiegenden Mängeln behaftet. Insbesondere ist bei diesem Verfahren die Verwendung von hohen Mengen an organischen Amidinsalzen erforderlich, die im Zuge der Aufarbeitung störend wirken können. Im übrigen ist auch das Verfahren der DE-OS 2 623 694 mit dem Nachteil behaftet, daß sehr hohe Mengen an Selen bzw. Selenverbindungen eingesetzt werden müssen.

Es war daher Aufgabe der vorliegenden Erfindung, ein verbessertes Verfahren zur Herstellung von Urethanen aus aromatischen Nitroverbindungen, Alkoholen und Kohlenmonoxid zur Verfügung zu stellen, welches nicht mit den genannten Nachteilen der Verfahren des Standes der Technik behaftet ist, und welches insbesondere eine wesentliche Reduzierung der Menge an Selen bzw. Selenverbindung gestattet, wobei trotz der Reduzierung der Katalysatormenge eine möglichst quantitative Urethanbildung ermöglicht werden soll.

Diese Aufgabe konnte durch das nachstehend näher erläuterte erfindungsgemäße Verfahren gelöst werden, welches insbesondere dadurch gekennzeichnet ist, daß man als Katalysatoren-Komponente bicyclische Amidine in Abwesenheit von Säuren insbesondere von Carbonsäuren oder Phenolen mitverwendet. Es muß als überraschend bezeichnet werden, daß bei Verwendung von bicyclischen Amidinen ohne gleichzeitige Mitverwendung einer Carbonsäure oder eines Phenols eine Reduzierung der Menge an Selen bzw. Selenverbindung möglich ist, da aufgrund der Offenbarung der DE-OS 2 623 694 zu erwarten war, daß die gleichzeitige Mitverwendung eines Phenols oder einer Carbonsäure unbedingt erforderlich ist.

Gegenstand der vorliegenden Erfindung ist ein Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von Selen und/oder Selenverbindungen in einer Menge, die 0,1 bis 3 Gew.-% Selen, bezogen auf die als Ausgangsmaterial eingesetzte Nitroverbindung entspricht, sowie aromatische Aminoverbindungen und/oder aromatische Harnstoffverbindungen aufweisenden Katalysatorn Systemen, dadurch gekennzeichnet, daß man solche Katalysator-Systeme verwendet, welche frei von Carbonsäuren und Phenolen sind und bicyclische Amidine aufweisen.

Ausgangsverbindungen für das erfindungsgemäße Verfahren sind.

1. aromatische Nitroverbindungen wie z.B. Nitrobenzol, 1,3-Dinitrobenzol, o-Nitrotoluol, m-Nitrotoluol, p-Nitrotoluol, 2,4-Dinitrotoluol, 2,6-Dinitrotoluol, Nitronaphthaline, Nitroanthracene, Nitrobiphenylene und dgl.. Im allgemeinen weisen die erfindungsgemäß geeigneten Nitroverbindungen ein Molekulargewicht von 123—400, 1—3 aromatische Kerne, sowie 1—3 an aromatische Kerne gebundene Nitrogruppen, sowie gegebenenfalls weitere unter den Reaktionsbedingungen des erfindungsgemäßen Verfahrens inerte Substituenten auf. Zu den bevorzugten Nitroverbindungen für das erfindungsgemäße Verfahren gehören Nitrobenzol, sowie die genannten Dinitrotoluole. Beliebige Gemische der genannten Nitroverbindungen können selbstverständlich ebenfalls eingesetzt werden.

2. Aliphatische, cycloaliphatische oder araliphatische Alkohole, d.h. beliebige, unter den Reaktionsbedingungen ansonsten inerte organische Verbindungen mit mindestens einer aliphatisch, cycloaliphatisch oder araliphatisch gebundenen Hydroxylgruppe des Molekulargewichtsbereichs 32—311. Beispiele geeigneter Alkohole sind primäre, sekundäre oder tertiäre Alkohole wie z.B. Methanol, Äthanol, n-Propanol, iso-Propanol, die verschiedenen isomeren Butanole, Cyclohexylalkohol, Benzylalkohol, Hexylalkohol, Laurylalkohol, Cetylalkohol, u.dgl.. Vorzugsweise werden beim erfindungsgemäßen Verfahren einwertige Alkohole, besonders bevorzugt Äthanol, eingesetzt.

3. Gasförmiges Kohlenmonoxid.

Beim erfindungsgemäßen Verfahren werden Katalysatoren-Systeme eingesetzt, welche a) Selen bzw. eine Selenverbindung, b) ein bicyclisches Amidin und c) eine aromatische Aminoverbindung und/oder eine aromatische Harnstoffverbindung aufweisen.

Geeignete Katalysatoren-Komponenten a) sind entweder elementares Selen in beliebiger, vorzugsweise jedoch in metallischer Form oder anorganische Selenverbindungen wie z.B. Selendioxid oder Carbonylselenid (COSe). Prinzipiell denkbar ist auch die Verwendung von organischen Selenverbindungen, wie z.B. Dimethylselenid, Diphenylselenid u.dgl.. Elementares Selen und Selendioxid sind besonders bevorzugt.

Bei der Katalysatoren-Komponente b) handelt es sich vorzugsweise um bicyclische Amidine der Formel

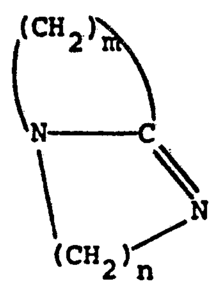

in welcher
m für eine ganze Zahl von 3 bis 5 und
n für eine ganze Zahl von 2 bis 4 stehen.

Bevorzugte derartige bicyclische Amidine sind z.B. 1,5-Diazabicyclo[4.3.0]-non-5-en oder 1,8-Diazabicyclo [5.4.0]-undecen-7.

Bei der Katalysatoren-Komponente c) handelt es sich um beliebige aromatisch gebundene primäre Aminogruppen und/oder aromatisch gebundene Harnstoffgruppen aufweisende organische Verbindungen, die neben den genannten Gruppen, insbesondere auch noch Nitrogruppen und Urethangruppen aufweisen können. Im allgemeinen handelt es sich bei der Komponente c) der erfindungsgemäß einzusetzenden Katalysatoren-Systeme um Verbindungen bzw. Gemische von Verbindungen, welche den Formeln

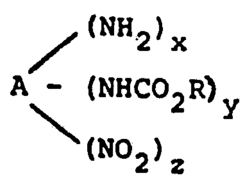

und/oder

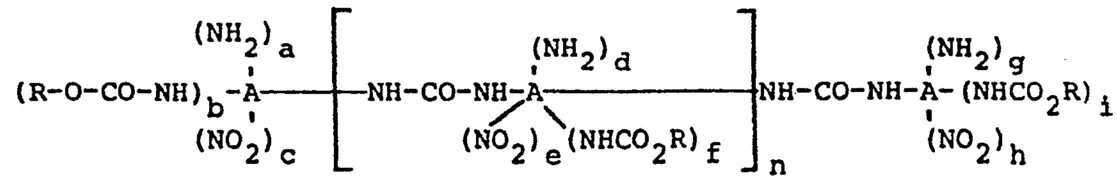

entsprechen.

In diesen Formeln bedeuten

x 1 oder 2,

y 0 oder 1,

z 0 oder 1, wobei die Summe x + y + z vorzugsweise 1 oder 2 beträgt,

a, b, c, d, e, f, g, h und i jeweils 0 oder 1, wobei die Summe a + b + c gleich der Summe g + h + e ist und 0, 1 oder 2 bedeutet, während die Summe d + e + f im Falle von a + b + c = 1 oder 2 einem um 1 verminderten Wert, d.h. 0 oder 1 darstellt, und im Falle von a + b + c = 0 ebenfalls für 0 steht,

n 0, 1, 2 oder 3, vorzugsweise jedoch 0 bedeutet,

A einen 1-, 2- oder 3-wertigen, vorzugsweise 1-oder 2-wertigen, gegebenenfalls $C_1$—$C_4$-Alkyl-substituierten aromatischen Kohlenwasserstoffrest, der im übrigen vorzugsweise dem aromatischen Kohlenwasserstoffrest der beim erfindungsgemäßen Verfahren einzusetzenden aromatischen Nitroverbindung entspricht und

R einen aliphatischen cycloaliphatischen oder araliphatischen Kohlenwasserstoffrest, mit im allgemeinen bis zu 18 Kohlenstoffatomen, der im übrigen vorzugsweise dem Kohlenwasserstoffrest der beim erfindungsgemäßen Verfahren einzusetzenden Alkoholkomponente entspricht.

Beispiele geeigneter Katalysatoren-Komponenten c) sind Anilin, o-, m- oder p-Toluidin, die isomeren Nitroaniline, die isomeren Diaminobenzole, N,N' - Diphenylharnstoff, N,N' - Bis(2 - methyl - 5 - nitro - phenyl) - harnstoff, N,N' - Bis(2 - methyl - 5 - äthoxycarbonylamino - phenyl) - harnstoff, N,N' - Bis - (2 - methyl - 5 - amino - phenyl) - harnstoff, 2 - Amino - 4 - nitrotoluol, 4 - Amino - 2 - nitrotoluol, 2 - Amino - 4 - äthoxycarbonylaminotoluol, 4 - Amino - 2 - äthoxycarbonylamino - toluol, 2,4 - Diaminotoluol, N,N' - Bis - (3 - nitro - 4 - methyl - phenyl) - harnstoff, N,N' - Bis - (2 - methyl - 5 - nitrophenyl) - harnstoff, N,N' - Bis - (3 - äthoxycarbonylamino - 4 - methylphenyl) - harnstoff, N,N' - Bis - (2 - methyl - 5 - äthoxycarbonylamino - phenyl) - harnstoff, N,N' - Bis - (3 - amino - 4 - methylphenyl) - harnstoff, N,N' - Bis - (2 - methyl - 5 - aminophenyl) - harnstoff, N - (3 - Nitro - 4 - methyl - phenyl) - N' - (2 - methyl - 5 - nitrophenyl) - harnstoff, N - (3 - Äthoxycarbonylamino - 4 - methylphenyl) - N' - (2 - methyl - 5 - äthoxycarbonylaminophenyl) - harnstoff, N - (3 - Amino - 4 - methylphenyl) - N' - (2 - methyl - 5 - aminophenyl) - harnstoff, N - (3 - Nitro - 4 - methyl - phenyl) - N' - (3 - äthoxycarbonylamino - 4 - methylphenyl) - harnstoff, N - (3 - Nitro - 4 - methylphenyl) - N' - (2 - methyl - 5 - äthoxycarbonylamino - phenyl) - harnstoff, N - (3 - Nitro - 4 - methylphenyl) - N' - (3 - amino - 4 - methyl - phenyl) - harnstoff, N - (3 - Nitro - 4 - methylphenyl) - N' - (2 - methyl - 5 - aminophenyl) - harnstoff, N - (2 - Methyl - 5 - nitrophenyl) - N' - (3 - äthoxycarbonylamino - 4 - methylphenyl) - harnstoff, N - (2 - Methyl - 5 - nitrophenyl) - N' - (2 - methyl - 5 - äthoxycarbonylamino - phenyl) - harnstoff,N - (2 - Methyl - 5 - nitrophenyl) - N' - (3 - Amino - 4 - methylphenyl) - harnstoff, N - (2 - Methyl - 5 - nitrophenyl) - N' - (2 - methyl - 5 - amino - phenyl) - harnstoff, N - (3 - Äthoxycarbonylamino - 4 - methylphenyl) - N' - (2 - methyl - 5 - aminophenyl) - harnstoff, N - (2 - Methyl - 5 - äthoxycarbonylamino - phenyl) - N' - (3 - amino - 4 - methylphenyl) - harnstoff, N' - (2 - Methyl - 5 - äthoxycarbonylaminophenyl) - N' - (2 - methyl - 5 - amino - phenyl) - harnstoff, sowie beliebige Gemische der beispielhaft genannten Verbindungen. Wie bereits dargelegt, werden vorzugsweise solche Verbindungen c) eingesetzt, die bezüglich ihres aromatischen Restes der beim erfindungsgemäßen Verfahren einzusetzenden aromatischen Nitroverbindung entsprechen. Bei Verwendung von Nitrobenzol wird demgemäß beispielsweise Anilin und/oder Diphenylharnstoff verwendet, während bei Verwendung von Nitrotoluol ein Toluoylamin und/oder ein Ditoluoylharnstoff zum Einsatz gelangt. Dementsprechend werden bei Verwendung zweiwertiger Nitroverbindungen beispielsweise von 2,4-Dinitrotoluol, die entsprechenden, zweifach substituierte Toluoylreste aufweisenden Verbindungen eingesetzt.

Höhere Homologe der beispielhaft aufgeführten Harnstoffe, d.h. mehrere Harnstoffeinheiten aufweisende Verbindungen können ebenfalls eingesetzt werden.

Bei der Durchführung des erfindungsgemäßen Verfahrens kommen die Reaktionspartner im allgemeinen in solchen Mengen zum Einsatz, daß für jede Nitrogruppe der als Ausgangsmaterial eingesetzten aromatischen Nitroverbindung zwischen 1 bis 50 vorzugsweise zwischen 5 bis 30 Hydroxylgruppen der Alkoholkomponente vorliegen. Das Kohlenmonoxid wird im allgemeinen im Überschuß eingesetzt.

Die Katalysator-Komponente a), d.h. das elementare Selen bzw. die Selenverbindung, welche auch auf einen geeigneten Träger, wie Kohlenstoff, Aluminiumoxid, Siliciumdioxid, Diatomeenerde, aktivierter Ton, Zeolith, Molekularsieben, Bariumsulfat, Kalziumcarbonat, Ionenaustauscherharzen und ähnlichen Materialen aufgebracht werden kann, wird in einer Menge eingesetzt, die 0,1 bis 3 Gew.-%

Selen, bezogen auf die als Ausgangsmaterial eingesetzte Nitroverbindung entspricht.

Die Katalysatoren-Komponente b), d.h. das bicyclische Amidin liegt im Reaktionsgemisch im allgemeinen in einer Menge von 1 bis 40 Mol.-% vorzugsweise 4 bis 20 Mol.-% bezogen auf als Ausgangsmaterial verwendete Nitroverbindung vor.

Die Katalysatoren-Komponente c) liegt im Reaktionsgemisch im allgemeinen in einer Menge von 1 bis 40 Mol.-%, vorzugsweise 4 bis 25 Mol.-%, bezogen auf als Ausgangsmaterial eingesetzte Nitroverbindung vor.

Das erfindungsgemäße Verfahren kann in Abwesenheit eines Lösungsmittels erfolgen, da der Alkohol als Lösungsmittel dient, jedoch kann auch ein Lösungsmittel verwendet werden. Beispiele für solche Lösungsmittel umfassen aromatische Lösungsmittel, wie Benzol, Toluol, Xylol usw., Nitrile, wie Acetonitril, Benzonitril, usw., Sulfone, wie Sulfolan, aliphatische halogenierte Kohlenwasserstoffe, wie 1,1,2-Trichlor-1,2,2-trifluoroäthan, aromatische halogenierte Kohlenwasserstoffe, wie Monochlorbenzol, Dichlorbenzol, Trichlorbenzol usw., Ketone, Ester und andere Lösungsmittel, wie Tetrahydrofuran, 1,4-Dioxan, 1,2-Dimethoxyäthan und ähnliches.

Die Reihenfolge der Zugabe der Ausgangsmaterialien und des Katalysatorsystems ist nicht beschränkt und kann wahlfrei je nach der Art der verwendeten Apparatur verändert werden. Beispielsweise wird eine Ausgangsmischung aus Alkohol, Selenkatalysator, Amin- und/oder Harnstoff-Verbindung, Amidin, und organischer Nitroverbindung in einen geeigneten druckbeständigen Reaktor, wie ein Autoklav, eingeführt, worauf weiter Kohlenmonoxid unter Druck eingebracht wird, wonach unter Erwärmung gerührt wird, bis die Urethanbildungsreaktion beendet ist. Kohlenmonoxid kann in den Reaktor entweder halbkontinuierlich oder kontinuierlich eingeleitet werden, während das beim Fortschreiten der Reaktion gebildete Kohlendioxid abgetrennt wird. Die Reaktion kann sowohl ansatzweise als auch halbkontinuierlich oder kontinuierlich durchgeführt werden. Das nach Beendigung der Reaktion im Überschuß anwesende Kohlenmonoxid kann durch Rezirkulation erneut verwendet werden.

Die Reaktionstemperatur wird im allgemeinen im Bereich von 80 bis 220°C, vorzugsweise 120 bis 200°C, gehalten. Obwohl die Reaktion bei höheren Reaktionstemperaturen schneller abläuft, besteht bei Temperaturen oberhalb 220°C die Neigung zu einer thermischen Zersetzung, wodurch die Ausbeute an Urethanprodukt verringert wird. Der Reaktionsdruck, d.h. der anfängliche Kohlenmonoxiddruck, liegt im allgemeinen im Bereich von 10 bis 300 bar, vorzugsweise 20 bis 150 bar. Die Reaktionszeit hängt von der Natur und Art der verwendeten Nitroverbindung, der Reaktionstemperatur, dem Reaktionsdruck, der Art und Menge des Katalysators und der Art der Apparatur ab, jedoch liegt sie im allgemeinen im Bereich von 5 Minuten bis 6 Stunden. Nach Bendigung der Reaktion wird die Reaktionsmischung der Abkühlung überlassen bzw. abgekühlt. Nach dem Ablassen des eingefüllten Gases wird die Reaktionsmischung einer bekannten Abtrennung, wie Filtration, Destillation oder einer anderen geeigneten Methode, unterworfen, um das gebildete Urethan von nicht-umgesetzten Materialien, Nebenprodukten, Lösungsmittel und Katalysator abzutrennen.

Die nach Abtrennung des Urethans zurückbleibende Reaktionsmischung enthält die Aminoverbindungen und Harnstoffverbindungen sowie nicht abgetrennte Urethananteile. Durch Rückführung dieser Rückstände zur Wiederverwendung erübrigt es sich, in einem weiteren Ansatz erneut irgendwelche Amino- und/oder Harnstoffverbindungen dem Reaktionssystem zuzuführen. Diese Methode der Wiederverwendung der Rückstände ist insbesondere bei kontinuierlicher Arbeitsweise von Vorteil.

Bei der Durchführung des erfindungsgemäßen Verfahrens sollte auf Wasserausschluß geachtet werden, da trotz des Zusatzes der Katalysatoren-Komponenten c) eine teilweise hydrolytische Spaltung der erfindungsgemäßen Verfahrensprodukte bei Anwesenheit von Wasser nicht ausgeschlossen werden kann.

Der erfindungswesentliche Punkt ist beim erfindungsgemäßen Verfahren in der aus DE-OS 2 623 694 bereits bekannten Mitverwendung von bicyclischen Amidinen zu sehen, wobei jedoch im Unterschied zu der genannten Vorveröffentlichung beim erfindungsgemäßen Verfahren das Amidin in "freier" Form, d.h. ohne gleichzeitige Mitverwendung von Carbonsäuren oder Phenolen zum Einsatz gelangt. Dank dieses Promotors ist bei ausgezeichneter katalytischer Wirksamkeit eine ganz wesentliche Verringerung der Selenmenge im Katalysatoren-System möglich. Die erfindungsgemäße Sonderstellung des bicyclischen Amidins wird dadurch deutlich, daß andere tertiäre Amine, wie z.B. Diazabicyclo[2.2.2]octan oder Pyridin zu einer ganz wesentlichen Verschlechterung der Wirksamkeit des Katalysatoren-Systems führen. Die wesentliche Rolle des "freien" Amidins ist schließlich völlig überraschend. Im Augenblick steht für diese überraschende Wirkung dieser Verbindungen keine plausible Erklärung zur Verfügung, zumal die "freien" Amidine ausdrücklich als unwirksam beschrieben wurden.

Die erfindungsgemäßen Verfahrensprodukte stellen wertvolle Zwischenprodukte zur Herstellung von Schädlingsbekämpfungsmitteln oder zur Herstellung von Polyurethanen dar. Insbesondere eignen sich die erfindungsgemäßen Verfahrensprodukte als Ausgangsmaterialien zur Herstellung der entsprechenden Isocyanate bzw. Polyisocyanate durch an sich bekannte Abspaltung der Alkoholkomponente.

Die nachfolgenden Beispiele veranschaulichen die Erfindung, ohne sie jedoch einzuschränken. In den Beispielen wurden alle Reaktionen in einem mit einem Rührer versehenen Autoklaven aus rostfreiem Stahl durchgeführt. Die in den Beispielen angegebenen Ausbeuten wurden jeweils aus den

Ergebnissen der Gaschromatographie und Flüssigkeitschromatographie errechnet. 1,5-Diazabicyclo[4.3.0]-non-5-en ist in folgenden als DBN, 1,8-Diazabicyclo[5.4.0]-undecen-7 als DBU bezeichnet.

Beispiel 1

17,50 g Nitrobenzol, 2,10 g DBU, 0,14 g metallisches Selen, 2,66 g Anilin und 140 g abs. Äthanol wurden in eine 0,7 l Autoklaven eingebracht. Die Luft im Autoklaven wurde durch Stickstoffgas und dann durch Kohlenmonoxid ersetzt. Anschließend wurde Kohlenmonoxid in den Autoklaven unter Druck eingeleitet, bis der Anfangsdruck 70 bar bei Raumtemperatur erreichte. Unter Rühren wurde das System auf 170°C aufgeheizt und für weitere 20 Minuten (Reaktionszeit) bei 170°C gerührt. Während dieser Zeit wurde ein Druckabfall beobachtet. Die Reaktionslösung wurde auf Raumtemperatur abgekühlt, und das Gas im Reaktionssystem durch Stickstoff ersetzt. Anschließend wurde die Reaktionslösung aus dem Autoklaven abgelassen. Nach Abtrennung des festen Selens durch Filtration wurde das erhaltene Filtrat einer gaschromatographischen Analyse unterworfen, die ergab, daß das Nitrobenzol zu 100% umgewandelt war und das Filtrat 23,96 g Äthyl-N-phenylcarbamat enthielt. Vom eingebrachten Anilin blieben 2,01 g in der Reaktionslösung.

Vergleichsbeispiel 1a

Beispiel 1 wurde mit dem organischen Salz, bestehend aus 2,10 g DBU und 0,84 g Essigsäure wiederholt. Das Nitrobenzol war zu 52,7% umgewandelt. Das Filtrat enthielt 15,2 g Äthyl-N-phenylcarbamat und 0,43 g Anilin.

Vergleichsbeispiel 1b

Beispiel 1 wurde ohne Anilin-Zusatz wiederholt. Das Nitrobenzol war zu 26,1% umgewandelt, das Filtrat enthielt 2,12 g Äthyl-N-phenylcarbamat.

Vergleichsbeispiel 1c

Beispiel 1 wurde mit 1,54 g Diazabicyclo[2.2.2]octan anstatt DBU wiederholt. Das Nitrobenzol war zu 13,3% umgewandelt. Das Filtrat enthielt 4,0 g Äthyl-N-phenyl-carbamat und 1,18 g Anilin.

Vergleichsbeispiel 1d

Beispiel 1 wurde mit 1,09 g Pyridin anstatt DBU wiederholt. Das Nitrobenzol war nur zu 3,6% umgewandelt. Das Filtrat enthielt 0,56 g Äthyl-N-phenylcarbamat und 2,23 g Anilin.

Beispiel 2

Beispiel 1 wurde mit 1,71 g DBN anstatt DBU wiederholt. Das Nitrobenzol war zu 98,9% umgewandelt. Das Filtrat enthielt 25,00 g Äthyl-N-phenylcarbamat und 0,77 g Anilin.

Beispiel 3

Beispiel 1 wurde mit 1,05 g DBU wiederholt. Das Nitrobenzol war quantitativ umgewandelt, im Filtrat wurden 23,7 g Äthyl-N-phenylcarbamat und 1,22 g Anilin nachgewiesen.

Beispiel 4

Entsprechend Beispiel 1 wurden 6 g N,N'-Diphenylharnstoff anstatt Anilin zugesetzt. Das Reaktionssystem wurde 1 Stunde bei 170°C gerührt und entsprechend Beispiel 1 aufgearbeitet. Das Filtrat enthielt 29,5 g Äthyl-N-phenyl-carbamat, das Nitrobenzol war quantitativ umgewandelt.

Beispiel 5

17,5 g Nitrobenzol, 1,05 g DBU, 0,7 g metallisches Selen, 2,66 g Anilin und 140 g abs. Äthanol wurden entsprechend Beispiel 1 30 Minuten bei 160°C gerührt. Die gaschromatographische Analyse wies eine quantitativ Umwandlung des Nitrobenzols aus. Das Filtrat enthielt 24,7 g Äthyl-N-phenylcarbamat und 0,31 g Anilin.

Beispiel 6

Beispiel 5 wurde mit 1 g Selendioxid statt metallischem Selen wiederholt. Das Nitrobenzol war quantitativ umgesetzt, daß Filtrat enthielt 23,6 g Äthyl-N-phenylcarbamat und 1,02 g Anilin.

Beispiel 7

25,46 g 2,4-Dinitrotoluol, 3,32 g DBU, 0,7 g metallisches Selen, 3,5 g 2,4-Diaminotoluol und 140 g abs. Äthanol wurden in einen 0,7 l Autoklaven eingebracht. Die Luft im Autoklaven wurde durch Stickstoffgas und dann durch Kohlenmonoxid ersetzt. Anschließend wurde Kohlenmonoxid in den Autoklaven unter Druck eingeleitet, bis der Anfangsdruck von 100 bar bei Raumtemperatur erreicht war. Das Reaktionssystem wurde aufgeheizt und 2 Stunden bei 170°C gerührt. Die nach der Flüssigkeitschromatographie vorgenommene Analyse des von Selen abgetrennten Filtrats ergab eine quantitative

Umsetzung des 2,4-Dinitroluols. Die Reactionslösung enthielt 29,7 g 2,4-Diäthoxycarbonylaminotoluol, 2,1 g 2-Nitro-4-äthoxycarbonylamino-toluol und 0,1 g 4-Nitro-2-äthoxycarbonylamino-toluol.

**Patentanspruch**

Verfahren zur Herstellung von Urethanen durch Umsetzung von aromatischen Nitroverbindungen mit aliphatischen, cycloaliphatischen oder araliphatischen Alkoholen und Kohlenmonoxid in Gegenwart von Selen und/oder Selenverbindungen in einer Menge, die 0,1 bis 3 Gew.-% Selen, bezogen auf die als Ausgangsmaterial eingesetzte Nitroverbindung entspricht, sowie aromatische Aminoverbindungen und/oder aromatische Harnstoffverbindungen aufweisenden Katalysator-Systemen, dadurch gekennzeichnet, daß man solche Katalysator-Systeme verwendet, welche frei von Carbonsäuren und Phenolen sind und bicyclische Amidine aufweisen.

**Revendication**

Procédé de fabrication d'uréthanes par réaction de composés nitrés aromatiques avec des alcools aliphatiques, cycloaliphatiques ou araliphatiques, et de l'oxyde de carbone en présence de sélénium et/ou de composés de sélénium en une quantité qui correspond à 0,1 — 3% en poids de sélénium par rapport au composé nitré utilisé comme matière première, ainsi que de systèmes de catalyseurs comportant des composés aminés aromatiques et/ou des composés d'urée aromatiques, caractérisé en ce qu'on utilise des systèmes de catalyseurs qui sont exempts d'acides carboxyliques et de phénols et qui comportent des amidines bicycliques.

**Claims**

Process for the preparation of urethanes by reacting aromatic nitro compounds with aliphatic, cycloaliphatic or araliphatic alcohols and carbon monoxide in the presence of selenium and/or selenium compounds in an amount corresponding to 0.1 to 3% by weight of selenium, relative to the nitro compound employed as the starting material, and of catalyst systems containing aromatic amino compounds and/or aromatic urea compounds, characterised in that such catalyst systems which are free from carboxylic acids and phenols and contain bicyclic amidines are used.